# EUROPEAN PATENT SPECIFICATION

(11) **EP 4 274 651 B1**
(45) Date of publication and mention of the grant of the patent: **05.11.2025**
(21) Application number: 22701756.3
(22) Date of filing: 05.01.2022
(51) Int. Cl.: A61M 25/00, A61M 25/01, A61M 1/36

(54) **COAXIAL CANNULA FOR USE WITH EXTRACORPOREAL MEMBRANE OXYGENATION SYSTEMS**
KOAXIALKANÜLE ZUR VERWENDUNG MIT EXTRAKORPORALEN MEMBRANOXYGENIERUNGSSYSTEMEN
CANULE COAXIALE À UTILISER AVEC LES SYSTÈMES D'OXYGÉNATION PAR MEMBRANE EXTRACORPORELLE

(30) Priority: 05.01.2021 US 202163133995 P
(43) Date of publication of application: 15.11.2023
(73) Proprietor: TC1 LLC, St. Paul, MN 55117 (US)
(72) Inventor: ANENDEN, Melissa, St. Paul, MN 55117 (US); HANSEN, J. Freddy, St. Paul, MN 55117 (US); HODGES, Bill, St. Paul, MN 55117 (US)
(74) Representative: Gualeni, Nadia
(86) International application number: PCT/US2022/011259
(87) International publication number: WO 2022/150346

(56) References cited:
- EP-A1- 1 980 288
- WO-A1-2019/241522
- US-A- 5 765 568
- US-A1- 2010 145 285
- US-A1- 2013 158 338
- US-A1- 2020 069 909

## Description

### BACKGROUND OF THE DISCLOSURE

### Field of the Disclosure

The present disclosure relates generally to extracorporeal membrane oxygenation systems, and more specifically, to a coaxial cannula for two-way blood flow for use in to extracorporeal membrane oxygenation systems.

### Background

Many types of cardiac assist devices have been developed for applications in which a patient's heart is incapable of providing adequate circulation, commonly referred to as heart failure or congestive heart failure. For example, a patient suffering from chronic heart failure may use a ventricular assist device or VAD that is implanted in the patient while awaiting a heart transplant or as a long term destination therapy. As another example, a patient suffering from acute heart failure may use an extracorporeal pump or circulatory support system that pumps blood out and back into a patient's body. Extracorporeal circulatory support systems may also be used perioperatively, for example, to direct blood through a patient while surgery is performed on the heart.

At least some extracorporeal circulatory support systems temporarily replace a patient's heart and lung functions by pumping blood around or bypassing the patient's heart and lungs. Such extracorporeal circulatory support systems will typically include an oxygenator, such as an extracorporeal membrane oxygenator or ECMO, to provide oxygen to the blood passing through extracorporeal circulatory support system.

At least some known extracorporeal circulatory support systems use cannulae that include single lumen cannulae at multiple insertion sites, high volume circuits and cannulae that are not capable of long term use. Multiple sites increase the risk of bleeding, vessel damage, infection, as well as pain and discomfort to the patient. Additionally, at least some known extracorporeal circulatory support system cannulae have a tendency to kink, or may cause the blood flow therethrough to result in blood damage.

Documents WO 2019/241522 A1 and US 2013/158338 A1 disclose known coaxial cannulas.

Accordingly, a need exists for extracorporeal circulatory support systems that provide a coaxial lumen cannula with improved blood flow.

### SUMMARY OF THE DISCLOSURE

The invention is as set forth in the appended claims.

The present disclosure is directed to a coaxial cannula assembly. The coaxial cannula assembly includes an infusion tube defining a return lumen and having a proximal end and a distal end, wherein the distal end of the infusion tube includes a plurality of infusion openings. The coaxial cannula also includes a drainage tube co-axially aligned with the infusion tube and having a proximal end and a distal end, wherein the distal end of the drainage tube includes a plurality of drainage openings and wherein a length of the drainage tube is less than a length of the infusion tube. A drainage lumen is defined by a space between the infusion tube and the drainage tube. The coaxial cannula also includes a flow router extending into and attached with the proximal end of the drainage tube and having a reservoir for receiving a fluid from the proximal end of the drainage tube. The infusion tube extends through the flow router, and wherein the diameter of the infusion tube is constant across the flow router as the infusion tube extends through the flow router from a proximal end of the flow router to a distal end of the flow router, and further wherein the infusion tube gradually tapers in diameter proximal the flow router.

### BRIEF DESCRIPTION OF THE DRAWINGS

FIG. 1 is an illustration of an extracorporeal circulatory support system connected to a patient's body.
FIG. 2 is a top perspective view of one embodiment of a coaxial cannula of the present invention.
FIG. 3A is a cross-sectional view of a portion of the coaxial cannula shown in FIG. 2 illustrating a flow router connected to various tubes.
FIG. 3B is an enlarged view of a distal end of the flow router shown in FIG. 3A.
FIG. 3C is an enlarged view of a proximal end of the flow router shown in FIG. 3A.
FIG. 4 is a cross-sectional end view of the flow router shown in FIG. 3A illustrating an infusion tube within a drainage tube.
FIG. 5A is a perspective cross-sectional view of the flow router illustrating one embodiment of a stator within the flow router.
FIG. 5B is a perspective cross-sectional view of the flow router illustrating another embodiment of a stator within the flow router.
FIG. 6 is a top view of a bend relief member circumscribing the infusion tube.
FIG. 7A is a top view of the coaxial cannula of FIG. 2 illustrating one embodiment of a stabilizing cuff proximal of the flow router.
FIG. 7B is a top view of the coaxial cannula of FIG. 2 illustrating a second embodiment of a stabilizing cuff proximal of the flow router.
FIG. 7C is a top view of the coaxial cannula of FIG. 2 illustrating a third embodiment of a stabilizing cuff proximal of the flow router.
FIG. 8 is a perspective view of one embodiment of the distal end of the infusion tube.
FIG. 9 is a perspective view of a second embodiment of the distal end of the infusion tube.
FIG. 10 is a perspective view of a third embodiment of the distal end of the infusion tube.
FIG. 11 is a perspective view of a fourth embodiment of the distal end of the infusion tube.
FIG. 12A is a perspective view of the coaxial cannula of FIG. 2 illustrating drainage slots at the distal end of the drainage tube.
FIG. 12B is a cross-sectional end view of the drainage slots shown in FIG. 12A.

### DETAILED DESCRIPTION OF THE DISCLOSURE

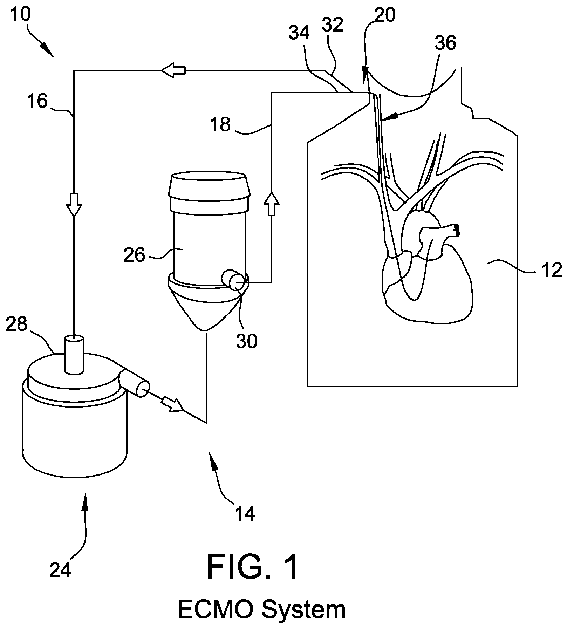

Referring now to the drawings, FIG. 1 is an illustration of an extracorporeal mechanical circulatory support system 10 connected a patient's 12 vasculature. The extracorporeal mechanical circulatory support system 10 includes a blood pump assembly 14, an inflow or first conduit 16, an outflow or second conduit 18, a coaxial tube cannula 20, a controller (not shown), and a power supply (not shown).

The blood pump assembly 14 includes a blood pump 24, an extracorporeal membrane oxygenator (ECMO) 26, and an inlet 28 and an outlet 30 for connection of flexible conduits thereto. The blood pump assembly 14 may include any suitable type of pump that enables the blood pump assembly 14 to function as described herein, including, for example and without limitation, an axial rotary pump and a centrifugal rotary pump. The ECMO 26 includes an oxygenator membrane (not shown) configured to increase the oxygen concentration and/or decrease the carbon dioxide concentration of blood pumped through the blood pump assembly 14. The oxygenator membrane may include any suitable type of oxygenator membrane that enables the blood pump assembly 14 to function as described herein including, for example and without limitation, fiber bundles. In some embodiments, the extracorporeal mechanical circulatory support system 10 also includes a purge valve (not shown) to release air or other gasses present within the extracorporeal mechanical circulatory support system 10. The purge valve can be connected, for example, to the outflow conduit 18, or may be integrated within the ECMO 26 (e.g., at an outlet of the ECMO 26).

The blood pump assembly 14 is connected to the patient's vasculature through the inflow conduit 16 and the outflow conduit 18. More specifically, the inlet 28 of the blood pump assembly 14 is connected to the inflow conduit 16, and the outlet 30 of the blood pump assembly 14 is connected to the outflow conduit 18. Furthermore, the inflow conduit 16 is connected to an outlet 32 of coaxial cannula 20, and the outflow conduit 18 is connected to an inlet 34 of coaxial cannula 20. The coaxial cannula 20 has substantial placement flexibility that allows the coaxial cannula 20 to be placed in a patient at various vascular insertion sites and depths. The coaxial cannula 20 is designed for insertion into the internal jugular vein 36 and placement above or below the right atrium and therefore will not typically cross the heart. Accordingly, the coaxial cannula 20 is less intrusive than cannulae that cross the heart. However, the coaxial cannula 20 can be used to cross the heart in certain applications. The coaxial cannula 20 is adapted for use with an introducer (not shown) that extends through the cannula and helps the user to properly place the coaxial cannula 20 in the correct position and depth in the body of the patient 12.

It will be understood that the illustrated connections to the patient's vasculature are for illustrative purposes only, and that the blood pump assembly 14 may be connected to the patient's vasculature in any other suitable manner that enables that extracorporeal mechanical circulatory support system 10 to function as described herein, including, for example and without limitation, veno-venous (VV) connections and veno-arterial (VA) connections..

The controller is communicatively coupled to the blood pump assembly 14, and is configured to control operation thereof. For example, the controller is configured to control operation (e.g., a speed) of the blood pump 24. The controller can generally include any suitable computer and/or other processing unit, including any suitable combination of computers, processing units and/or the like that may be communicatively coupled to one another (e.g., controller can form all or part of a controller network). Thus, controller can include one or more processor(s) and associated memory device(s) configured to perform a variety of computer-implemented functions (e.g., performing the methods, steps, calculations and/or the like disclosed herein). As used herein, the term "processor" refers not only to integrated circuits referred to in the art as being included in a computer, but also refers to a controller, a microcontroller, a microcomputer, a programmable logic controller (PLC), an application specific integrated circuit (ASIC), a digital signal processor (DSP), a field programmable gate array (FPGA), and other programmable circuits. Additionally, the memory device(s) of the controller may generally include memory element(s) including, but not limited to, non-transitory computer readable medium (e.g., random access memory (RAM)), computer readable non-volatile medium (e.g., a flash memory), a floppy disk, a compact disc-read only memory (CD-ROM), a magneto-optical disk (MOD), a digital versatile disc (DVD) and/or other suitable memory elements. Such memory device(s) can generally be configured to store suitable computer-readable instructions that, when implemented by the processor(s), configure the controller to perform various functions including, but not limited to, controlling components of the blood pump assembly 14 as described herein.

The power supply provides power to the blood pump 24, controller, and other electrical components of the blood pump assembly 14, and may generally include any suitable power supply that enables the extracorporeal mechanical circulatory support system 10 to function as described herein. The controller and power supply may be external to the blood pump assembly 14, or all or part of the controller and/or the power supply 22 may be incorporated within the blood pump assembly 14 in other embodiments.

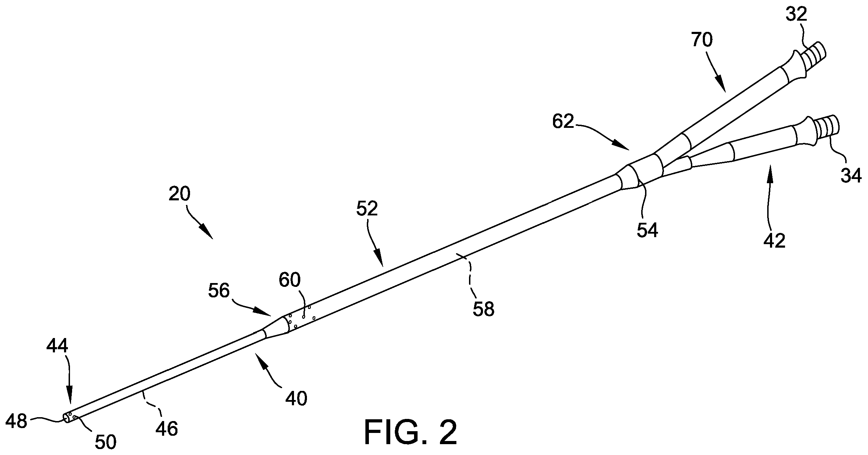

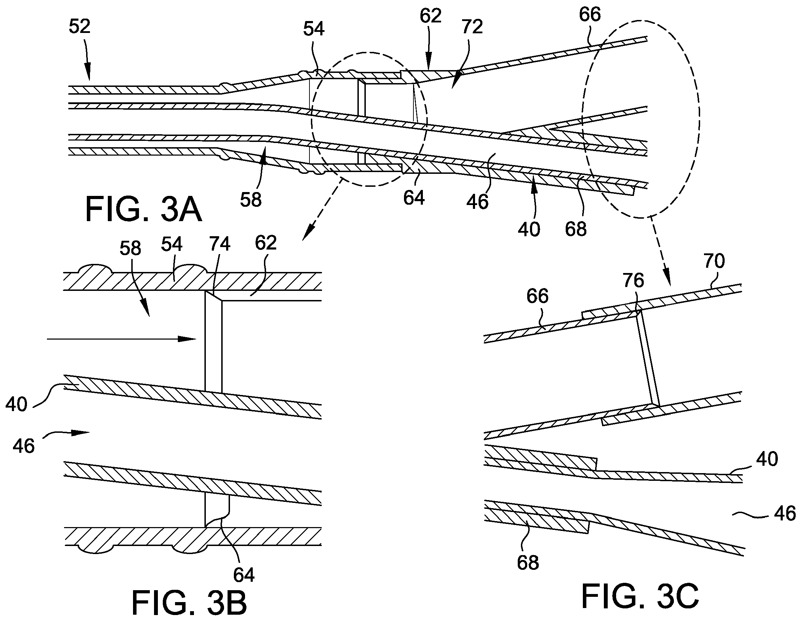

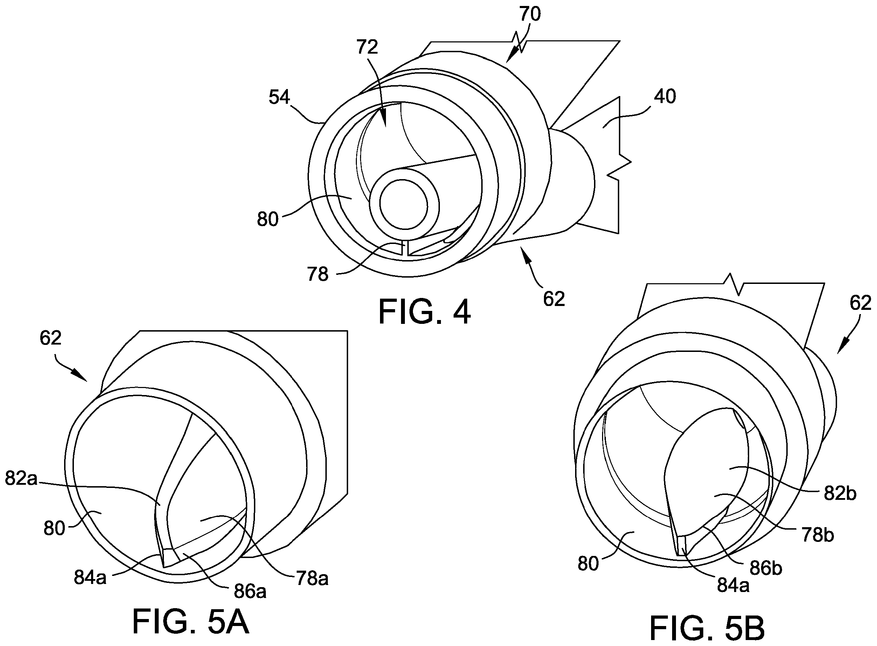

FIG. 2 is a top perspective view of one embodiment of a coaxial cannula 20. FIG. 3A is a cross-sectional view of a portion of the coaxial cannula 20 illustrating a flow router 62 connected to various tubes. FIG. 3B is an enlarged view of a distal end of the flow router 62. FIG. 3C is an enlarged view of a proximal end of the flow router 62. FIG. 4 is a cross-sectional end view of the flow router 62 illustrating an infusion tube 40 within a drainage tube 52. FIG. 5A is a perspective cross-sectional view of the flow router 62 illustrating one embodiment of a stator 78 within the flow router 62. FIG. 5B is a perspective cross-sectional view of the flow router 62 illustrating another embodiment of a stator 78 within the flow router 62.

The coaxial cannula 20 includes an infusion tube 40 having a proximal end 42, a distal end 44, and defining an internal or return lumen 46 extending between ends 42 and 44. The distal end 44 includes an end return aperture 48 and a plurality of infusion openings 50 defined through the infusion tube 40 and in flow communication with the return lumen 46. The coaxial cannula 20 also includes a drainage tube 52 that is coaxial with the infusion tube 40 and includes a proximal end 54, a distal end 56, and a drainage lumen 58 extending between ends 54 and 56. The distal end 56 of the drainage tube 52 is fixedly attached to the infusion tube 40 and includes a plurality of drainage openings 60 defined through the drainage tube 52 and in flow communication with the drainage lumen 58. As best shown in FIG. 3A, the infusion tube 40 is located within the drainage lumen 58 of the drainage tube 52 such that blood flowing through the drainage lumen 58 flows around the infusion tube 40. Specifically, the return lumen 46 includes a first cross-sectional diameter that is smaller than the drainage lumen 58 such that a predetermined ratio of the return lumen 46 to the drainage lumen 58 is defined. More specifically, the ratio is designed to reduce the pressure inside the drainage lumen 58 by increasing the cross-sectional area of the drainage lumen 58 with respect to the return lumen 46 as compared to at least some known coaxial cannulae.

A flow router 62 includes a distal end 64, a drainage proximal end 66, and an infusion proximal end 68. The distal end 64 is attached to the proximal end 54 of the drainage tube 52, the proximal drainage end 66 is connected to a proximal drainage tube 70, and the infusion tube 40 extends through the flow router 62 and exits the infusion proximal end 68. The flow router 62 also includes a reservoir 72 for receiving fluid from the proximal end 54 of the drainage tube 52, wherein the infusion tube 40 extends through the flow router 62 and does not connect with the drainage tube 52 at the flow router 62, and wherein the infusion tube 40 remains substantially coaxial with the drainage tube 52 throughout the length of the drainage tube 52.

Referring now to FIGs. 3B and 3C, the distal end 64 of the flow router 62 includes a transition feature 74 at the transition from the drainage tube 52 to the flow router 62. Similarly, the drainage proximal end 66 includes a transition feature 76 at the transition from the flow router 62 to the proximal drainage tube 70. The transition features 74 and 76 improve hemodynamics and hemocompatibility by allowing for a smooth transition between components that is free of a stepped transition, which may cause turbulence within the blood flow. As illustrated, transition features 74 and 76 are rounded or slanted surfaces that taper the thickness of the flow router 62 at the distal end 64 and the drainage proximal end 66.

Still referring to FIGs. 3A and 3C, it can be seen that infusion tube 40 includes a constant cross-sectional diameter throughout the length of the drainage tube 52 and throughout the length of the flow router 62. When the infusion tube 40 exits the flow router 62, the infusion tube 40 begins to taper outward into a larger diameter in order to match the diameter of the outflow conduit 18.

Referring now to FIGs. 4, 5A, and 5B, the flow router 62 includes a stator 78 that is used to smoothly direct blood flow from the drainage tube 52 around the infusion tube 40 within the reservoir 72 of the flow router 62. Specifically, the flow router 62 includes an interior surface 80 that defines reservoir 72 and the stator 78 extends from the interior surface 80 into the reservoir 72. The stator 78 includes a top surface 82, a distal tip 84, and a pair of sidewalls 86 that taper outward in a proximal direction from the tip 84. The sidewalls 86 may have any constant or varying height that enables operation of the stator 78 as described herein. Further, the sidewalls 86 meet at the tip 84 at any angle that enables operation of the stator 78 as described herein.

In operation, the infusion tube 40 is fixed to the top surface 82 and the tip 84 and the sidewalls 86 guide the blood flow around the infusion tube 40 and into the proximal drainage tube 70. Additionally, although only a single stator 78 is shown, the flow router 62 may include a plurality of stators 78 circumferentially spaced about interior surface 80. As such, the stator/s 78 guide the blood flow around the infusion tube 40 and into the proximal drainage tube 70 to reduce recirculation within reservoir 72. Furthermore, the stator/s 78 are positioned within the flow router 62 where the infusion tube 40 diverts to the side and the cross-section of the reservoir 72 available for drainage flow increases. As such, the stator/s 78 occupy space within the reservoir 72 to maintain a relatively constant pressure. FIG. 5A illustrates one embodiment of a stator 78a where the distal portions of the sidewalls 86a and top surface 82a blend into the interior surface 80 of the flow router 62. FIG. 5B illustrates an embodiment of a stator 78b where the sidewalls 86b and top surface 82b do not blend into the interior surface 80.

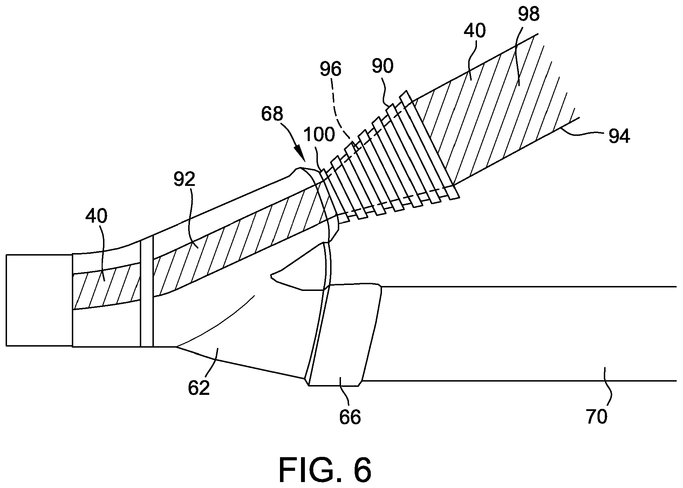

FIG. 6 is a top view of a bend relief member 90 circumscribing the infusion tube 40. The infusion tube 40 includes a distal portion 92 having a first diameter, a proximal portion 94 having a second diameter larger than the first diameter, and a tapered portion 96 between the proximal portion 94 and the distal portion 92. As described herein, the distal portion 92 extends through the flow router 62 and exits out the infusion proximal end 68 of the flow router 62 such that the tapered portion 96 begins proximal of the flow router 62. A bend relief member 90 may be positioned around at least the tapered portion 96 of the infusion tube 40 to prevent or reduce bending and kinking (a reduction in cross-sectional area) of the infusion tube 40 at the transition between the distal portion 92 and the tapered portion 96. The bend relief member 90 is a flexible material that may include metallic braiding for additional structural support. Additionally, the infusion tube 40 is shown in FIG. 6 as including braiding 98 for additional structural support. Such braiding 98 is not limited to embodiments that include the bend relief member 90, and may be included on all embodiments described herein. Furthermore, a distal end 100 of the bend relief member 90 may be connected to the infusion proximal end 68 of the flow router 62 to ensure the bend relief member covers the transition between the distal portion 92 and the tapered portion 96 of the infusion tube 40. The infusion proximal end 68 of the flow router 62 may include an engagement feature (not shown) to secure the bend relief member 90 in place.

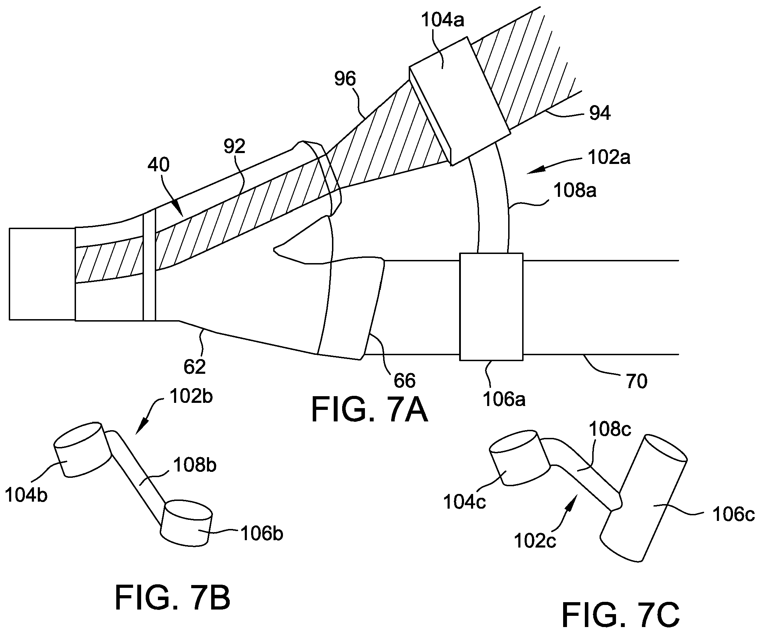

FIG. 7A is a top view of the coaxial cannula 20 illustrating one embodiment of a stabilizing cuff 102a proximal of the flow router 62. Stabilizing cuff 102a includes a first cuff 104a positioned around infusion tube 40 at the transition from the tapered portion 96 to the proximal portion 94. Stabilizing cuff 102a also includes a second cuff 106a positioned around proximal drainage tube 70 and spaced from flow router 62. A bridge member 108a extends between cuffs 104a and 106a and limits movement of the infusion tube 40 relative to the proximal drainage tube 70. Stabilizing cuff 102a provides support to the infusion tube 40 proximal the flow router 62 and may be used instead of bend relief member 90. Specifically, stabilizing cuff 102a is made from a stiff material and prevents or reduces bending and kinking (a reduction in cross-sectional area) of the infusion tube 40. Furthermore, the stabilizing cuff 102a may be used to maintain a predetermined angle between the infusion tube 40 and the proximal drainage tube 70 proximal the flow router 62.

FIG. 7B is a top view of the coaxial cannula 20 illustrating a second embodiment of a stabilizing cuff 102b proximal of the flow router 62. Stabilizing cuff 102b includes a first cuff 104b positioned around infusion tube 40 at the transition from the tapered portion 96 to the proximal portion 94. Stabilizing cuff 102b also includes a second cuff 106b positioned around flow router 62 and/or a portion of proximal drainage tube 70 adjacent to flow router 62. As such, the first cuff 104b and the second cuff 106b are offset from each other. A bridge member 108b extends between cuffs 104b and 106b and limits movement of the infusion tube 40 relative to the proximal drainage tube 70. Stabilizing cuff 102b provides support to the infusion tube 40 proximal the flow router 62 and may be used instead of bend relief member 90. Specifically, stabilizing cuff 102b is made from a stiff material and prevents or reduces bending and kinking (a reduction in cross-sectional area) of the infusion tube 40. Furthermore, the stabilizing cuff 102b may be used to maintain a predetermined angle between the infusion tube 40 and the proximal drainage tube 70 proximal the flow router 62.

FIG. 7C is a top view of the coaxial cannula 20 illustrating a third embodiment of a stabilizing cuff 120c proximal of the flow router 62. Stabilizing cuff 102c includes a first cuff 104c positioned around infusion tube 40 at the transition from the tapered portion 96 to the proximal portion 94. Stabilizing cuff 102c also includes a second cuff 106c positioned around flow router 62 and/or a portion of proximal drainage tube 70 adjacent to flow router 62. As such, the first cuff 104c and the second cuff 106c are offset from each other. Furthermore, the second cuff 106c is longer in length than the first cuff 104c to provide additional support. A bridge member 108c extends between cuffs 104c and 106c and limits movement of the infusion tube 40 relative to the proximal drainage tube 70. Stabilizing cuff 102c provides support to the infusion tube 40 proximal the flow router 62 and may be used instead of bend relief member 90. Specifically, stabilizing cuff 102c is made from a stiff material and prevents or reduces bending and kinking (a reduction in cross-sectional area) of the infusion tube 40. Furthermore, the stabilizing cuff 102c may be used to maintain a predetermined angle between the infusion tube 40 and the proximal drainage tube 70 proximal the flow router 62.

In another embodiment, a web (not shown) extends between the infusion tube 40 and the proximal drainage tube 70. The web may be an extension of the flow router 62 that is integral to the flow router 62 and secures around both the infusion tube 40 and the proximal drainage tube 70. Alternatively, the web is made from the same material as the infusion tube 40 and the proximal drainage tube 70.

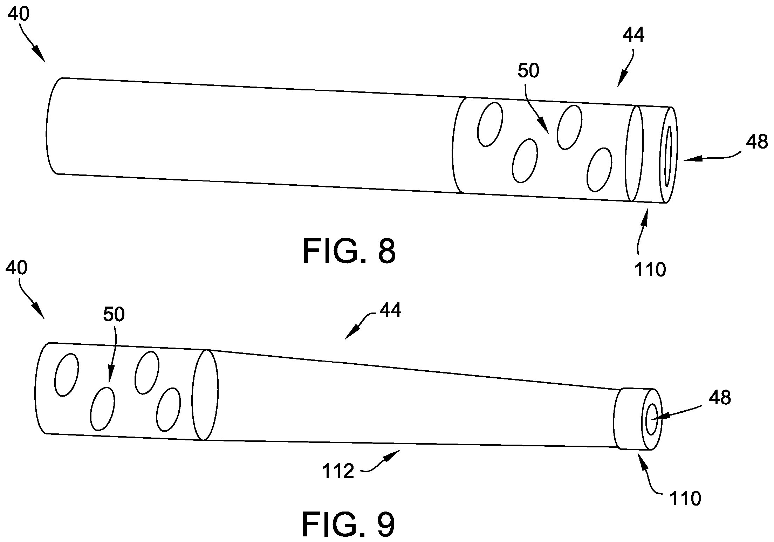

FIG. 8 is a perspective view of one embodiment of the distal end 44 of the infusion tube 40. As described herein, the infusion tube 40 is designed to allow blood to be infused into the main pulmonary artery of the patient 12. The distal end 44 of the infusion tube 40 includes the plurality of infusion openings 50 and the end return aperture 48. More specifically, the distal end 44 includes an end cap 110 positioned distal of the infusion openings 50 and in which end return aperture 48 is defined. In the illustrated embodiment, the distal end 44, including the end cap 110, includes constant inner and outer diameters. In another embodiment, the outer diameter of the end cap 110 is tapered for ease of insertion, but the inner diameter is constant. The end cap 110 is formed from a softer material than the infusion tube 40 for atraumatic insertion.

FIG. 9 is a perspective view of a second embodiment of the distal end 44 of the infusion tube 40. As described herein, the infusion tube 40 is designed to allow blood to be infused into the main pulmonary artery of the patient 12. The distal end 44 of the infusion tube 40 includes the plurality of infusion openings 50, a tapered portion 112 distal to the infusion openings 50, and the end return aperture 48. The tapered portion 112 allows a smooth transition between the cannula tip and the introducer (not shown). The tapered portion 112 also allows for more flexibility around the distal end 44 of the cannula 20 for tracking into position. Furthermore, the distal end 44 includes an end cap 110 positioned distal of the tapered portion 112 and in which end return aperture 48 is defined. In the illustrated embodiment, the distal end 44, including the end cap 110 and the tapered portion 112, includes a constant inner diameter. In another embodiment, the outer diameter of the end cap 110 is tapered for ease of insertion, but the inner diameter remains constant. The end cap 110 is formed from a softer material than the infusion tube 40 for atraumatic insertion.

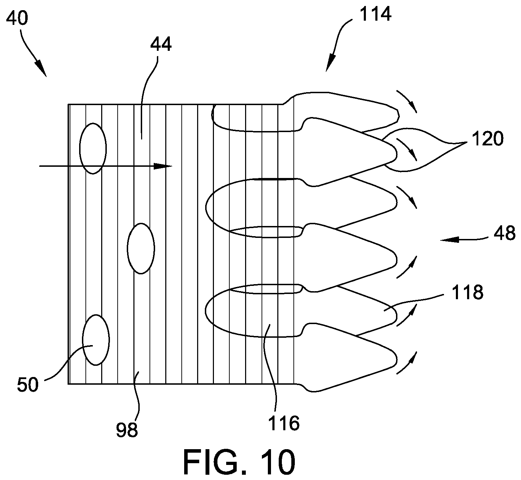

FIG. 10 is a perspective view of a third embodiment of the distal end 44 of the infusion tube 40. The distal end 44 includes the plurality of infusion openings 50 and a plurality of elongated flexible members 114 at the distal tip of the distal end 44 such that the distal tip is crenulated. Specifically, the distal end 44 illustrated in FIG. 10 includes elongate members 114 that define the end return aperture 48 rather than the end cap 110 shown in FIGs. 8 and 9. The elongate members 114 are circumferentially spaced about the perimeter of the infusion tube 40 and are separated by a plurality of infusion flow slots 116. More specifically, adjacent elongate members 114 are separated by an infusion flow slot 116. The infusion flow slots 116 are open-ended such that the distal tips 118 of the elongate members 114 are separated by the infusion flow slots 116. The configuration of the slots 116 and the elongate members 114 allows for blood to exit the distal end 44 even if the distal tip is abutted against a blood vessel or other structure within the patient 12. In operation, the infusion flow slots 116 allow for increased blood flow through slots 116 and through end return aperture 48, which allows for the infusion openings 50 to be smaller in size or fewer in number than if the distal end 44 did not include the infusion flow slots 116. More specifically, with the use of the infusion flow slots 116, the total cross-sectional area of the infusion openings 50 is less than the cross-sectional area of the infusion tube 40.

Additionally, as shown in FIG. 10, the distal tips 118 of the elongate members 114 taper toward the distal end. Specifically, each elongate member 114 includes a pair of circumferential sidewalls 120 that taper towards each other to form the distal tip 118. It is important to note that the tapering is circumferential such that the cross-sectional diameter of the elongate members 114 is constant. Such tapering allows the elongate members 114 to bend or flex inward during insertion of the coaxial cannula 20. During insertion, the distal tips 118 may engage a wall of the patient's vasculature and cause the engaging elongate member 114 to bend inward to facilitate an atraumatic insertion.

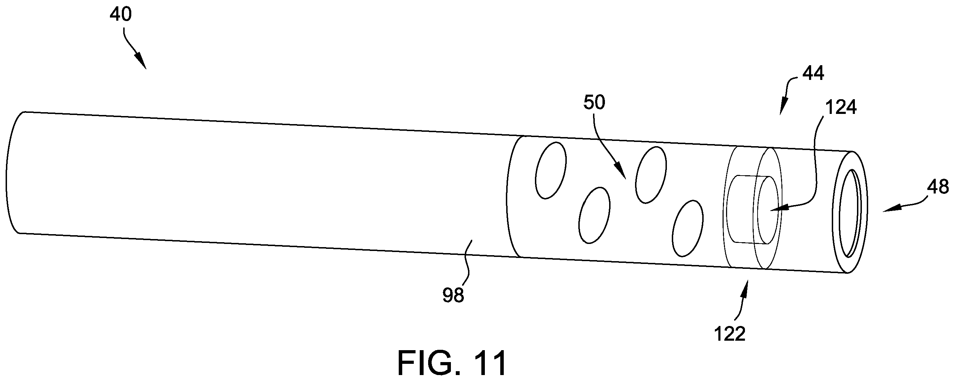

FIG. 11 is a perspective view of a fourth embodiment of the distal end 44 of the infusion tube 40. The illustrated distal end 44 includes the plurality of infusion openings 50 and a flow restriction member 122 positioned between the infusion openings 50 and the end return aperture 48. The flow restriction member 122 defines a lumen 124 with a cross-sectional area smaller than the cross-sectional area of the end return aperture 48. As such, in operation, an area of higher pressure will build up proximal of the flow restriction member 122. This high pressure area forces more blood flow through the infusion openings 50 than if the flow restriction member 122 were not present. The higher pressure and higher flow rate allow for the infusion openings 50 to be smaller in size or fewer in number than if the distal end 44 did not include the flow restriction member 122. More specifically, with the use of the flow restriction member 122, the total cross-sectional area of the infusion openings 50 is less than the cross-sectional area of the infusion tube 40.

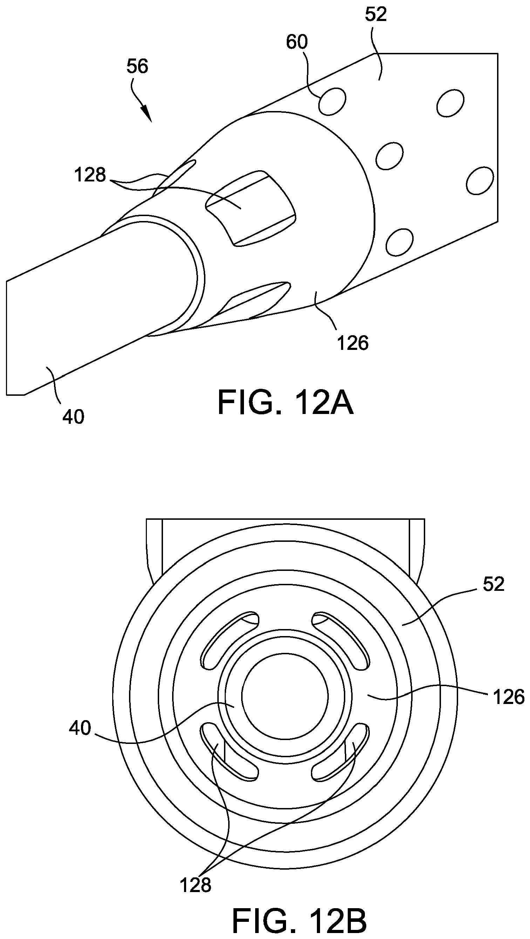

FIG. 12A is a perspective view of the coaxial cannula 20 illustrating the distal end 56 of the drainage tube 52. FIG. 12B is a cross-sectional end view of the distal end 56 of the drainage tube 52. The distal end 56 includes a tapered portion 126 that is connected to an exterior surface of the infusion tube 40. The tapered portion 126 is positioned distal to the plurality of drainage openings 60 formed in drainage tube 52. Furthermore, the tapered portion 126 includes a plurality of circumferentially-spaced slots 128 that enable the flow of blood therethrough. Although only four slots 128 are shown, tapered portion 126 may include any number of slots 128 that facilitates operation of cannula 20 as described herein. As shown in FIGs 12a and 12B, the slots 128 are oriented parallel to the axis of drainage tube 52 such that blood enters through the slots 128 in an axial direction rather than in a radial direction as does blood flow through the openings 60. The axially-oriented slots 128 allow for more efficient blood flow therethrough because the blood is not required to change direction as it is when flowing through the drainage openings 60. As such, the higher efficiency leads to a higher flow rate through the slots, which allows for the drainage openings 60 to be smaller in size and/or fewer in number than if the distal end 56 did not include the slots 128. More specifically, with the use of the slots 128, the total cross-sectional area of the drainage openings 60 plus the cross-sectional area of the slots 128 is less than the cross-sectional area of the drainage tube 52.

Although the embodiments and examples disclosed herein have been described with reference to particular embodiments, it is to be understood that these embodiments and examples are merely illustrative of the principles and applications of the present disclosure. It is therefore to be understood that numerous modifications can be made to the illustrative embodiments and examples and that other arrangements can be devised without departing from the scope of the present disclosure as defined by the claims.

This written description uses examples to disclose the disclosure, including the best mode, and also to enable any person skilled in the art to practice the disclosure, including making and using any devices or systems and performing any incorporated methods. The patentable scope of the disclosure is defined by the

## Claims

1. A coaxial cannula assembly (20) comprising:
an infusion tube (40) defining a return lumen (46) and having a proximal end (42) and a distal end (44), wherein said distal end (44) of said infusion tube (40) includes a plurality of infusion openings (50), wherein said distal end (44) of said infusion tube (40) comprises a flow restriction member (122) positioned between the plurality of infusion openings (50) and an end return aperture (48) at a distal tip of the infusion tube (40);
wherein the flow restriction member (122) comprises a ring that defines a central lumen, and wherein the ring creates a proximal area of higher pressure in the return lumen (46) that increases a flow rate through the plurality of infusion openings (50) without preventing fluid flow to the end return aperture (48); and
a drainage tube (52) co-axially aligned with said infusion tube (40) and having a proximal end (70) and a distal end (56), wherein said distal end of said drainage tube (52) includes a plurality of drainage openings (60) and wherein a length of said drainage tube (52) is less than a length of said infusion tube (40);
wherein a drainage lumen (58) is defined by a space between said infusion tube (40) and said drainage tube (52).

2. The coaxial cannula assembly (20) of Claim 1, wherein the flow restriction member (122) is positioned distal of the distalmost one of the plurality of infusion openings (50).

3. The coaxial cannula assembly (20) of Claim 1, wherein the central lumen defines a cross-sectional area that is smaller than a cross-sectional area of the end return aperture (48).

4. The coaxial cannula assembly (20) of Claim 1, wherein a total cross-sectional area of the infusion openings (50) is less than a cross-sectional area of the infusion tube (40).

5. The coaxial cannula assembly (20) of Claim 1,
wherein said distal end (44) of said infusion tube (40) comprises a plurality of elongate members (114) proximate said infusion openings (50) and defining the end return aperture (48) at a distal tip of the infusion tube (40), and wherein said plurality of elongate members (114) define open-ended axially elongated blood flow slots therebetween.

6. The coaxial cannula assembly (20) of Claim 5, wherein the plurality of elongate members (114) is evenly spaced about a circumference of the distal end (44) of the infusion tube (40).

7. The coaxial cannula assembly (20) of Claim 6, wherein adjacent ones of the plurality of elongate members (114) are separated by a single open-ended axially extending blood flow slot.

8. The coaxial cannula assembly (20) of Claim 5, wherein each of the plurality of elongate members (114) includes a pair of sidewalls that taper together at the distal tip.

9. The coaxial cannula assembly (20) of Claim 8, wherein the pair of sidewalls taper together at the distal tip in a circumferential direction such that the cross-sectional diameter of the elongate members (114) is constant.

10. The coaxial cannula assembly (20) of Claim 1, further comprising
a flow router (62) extending into and attached with said proximal end (54) of said drainage tube (70) and having a reservoir (72) for receiving a fluid from said proximal end of said drainage tube (70), wherein said flow router comprises a stator (78, 78a, 78b) extending into said reservoir and connected to said infusion tube (40), wherein said stator is configured to guide blood flow around said infusion tube (40).

11. The coaxial cannula (20) assembly of Claim 10, wherein the stator comprises a top surface (82), a distal tip (84), and a pair of sidewalls (86).

12. The coaxial cannula (20) assembly of Claim 11, wherein the sidewalls taper together to form the distal tip.

13. The coaxial cannula (20) assembly of Claim 11, wherein distal portions of the sidewalls converge toward the interior surface.

14. The coaxial cannula (20) assembly of Claim 10, wherein the infusion tube (40) is coupled to the top surface of the stator.

15. The coaxial cannula (20) assembly of Claim 10, wherein the stator is positioned within the reservoir such that a relatively constant pressure is maintained within the reservoir and wherein the stator is configured to guide blood flow around the infusion tube (40) and into the drainage tube (52) to reduce recirculation within the reservoir.

## Patentansprüche

1. Koaxialkanülenanordnung (20), umfassend:
ein Infusionsschlauch (40), der ein Rücklumen (46) definiert und ein proximales Ende (42) sowie ein distales Ende (44) aufweist, wobei das distale Ende (44) des Infusionsschlauchs (40) eine Mehrzahl von Infusionsöffnungen (50) umfasst, wobei das distale Ende (44) des Infusionsschlauchs (40) ein Strömungsbegrenzungselement (122) umfasst, das zwischen der Mehrzahl von Infusionsöffnungen (50) und einer Endrücköffnung (48) an einer distalen Spitze des Infusionsrohrs (40) angeordnet ist;
wobei das Strömungsbegrenzungselement (122) einen Ring umfasst, der ein Zentrallumen definiert, und wobei der Ring einen proximalen Bereich mit höherem Druck in dem Rücklumen (46) erzeugt, der einen Durchflussrate durch die Mehrzahl von Infusionsöffnungen (50) erhöht, ohne den Flüssigkeitsstrom zu der Rücköffnung (48) zu verhindern; und ein Drainagerohr (52), das koaxial mit dem Infusionsrohr (40) ausgerichtet ist und ein proximales Ende (70) sowie ein distales Ende (56) aufweist,
wobei das distale Ende des Drainagerohrs (52) eine Mehrzahl von Drainageöffnungen (60) umfasst und wobei eine Länge des Drainagerohrs (52) kleiner als eine Länge des Infusionsrohrs (40) ist;
wobei ein Drainagelumen (58) durch einen Raum zwischen dem Infusionsschlauch (40) und dem Drainagerohr (52) definiert wird.

2. Koaxialkanülenanordnung (20) nach Anspruch 1, wobei das Strömungsbegrenzungselement (122) distal von der distalsten der Mehrzahl von Infusionsöffnungen (50) angeordnet ist.

3. Koaxialkanülenanordnung (20) nach Anspruch 1, wobei das Zentrallumen einen Querschnittsbereich definiert, die kleiner als einen Querschnittsbereich der Rücköffnung (48) ist.

4. Koaxialkanülenanordnung (20) nach Anspruch 1, wobei ein Gesamtquerschnittsbereich der Infusionsöffnungen (50) kleiner als ein Querschnittsbereich des Infusionsschlauchs (40) ist.

5. Koaxialkanülenanordnung (20) nach Anspruch 1,
wobei das distale Ende (44) des Infusionsschlauchs (40) eine Mehrzahl von länglichen Elementen (114) in der Nähe der Infusionsöffnungen (50) umfasst, die die Endrücköffnung (48) an einer distalen Spitze des Infusionsschlauchs (40) definiert, und wobei die Mehrzahl von länglichen Elementen (114) dazwischen axial längliche, an den Enden offene Blutströmungsschlitze definieren.

6. Koaxialkanülenanordnung (20) nach Anspruch 5, wobei die Mehrzahl von länglichen Elementen (114) gleichmäßig beabstandet um einen Umfang des distalen Endes (44) des Infusionsschlauchs (40) angeordnet sind.

7. Koaxialkanülenanordnung (20) nach Anspruch 6, wobei angrenzende längliche Elemente (114) der Mehrzahl von länglichen Elementen durch einen einzigen an den Enden offenen Blutströmungsschlitz, der sich axial erstreckt, voneinander getrennt sind.

8. Koaxialkanülenanordnung (20) nach Anspruch 5, wobei jedes der Mehrzahl von länglichen Elementen (114) ein Paar von Seitenwänden umfasst, die sich gemeinsam an der distalen Spitze verjüngen.

9. Koaxialkanülenanordnung (20) nach Anspruch 8, wobei sich das Paar von Seitenwänden an der distalen Spitze in Umfangsrichtung verjüngen, sodass der Querschnittsdurchmesser der länglichen Elemente (114) konstant ist.

10. Koaxialkanülenanordnung (20) nach Anspruch 1, ferner umfassend
einen Strömungsverteiler (62), der sich in das proximale Ende (54) des Drainagerohrs (70) erstreckt und daran befestigt wird und ein Reservoir (72) zum Aufnehmen einer Flüssigkeit von dem proximalen Ende des Drainagerohrs (70) aufweist, wobei der Strömungsverteiler einen Stator (78, 78a, 78b) umfasst, der sich in das Reservoir erstreckt und mit dem Infusionsschlauch (40) verbunden ist, wobei der Stator dazu konfiguriert ist, die Blutströmung um das Infusionsschlauch (40) zu führen.

11. Koaxialkanülenanordnung (20) nach Anspruch 10, wobei der Stator eine obere Fläche (82), eine distale Spitze (84) und ein Paar von Seitenwänden (86) umfasst.

12. Koaxialkanülenanordnung (20) nach Anspruch 11, wobei sich die Seitenwände verjüngen, um die distale Spitze zu bilden.

13. Koaxialkanülenanordnung (20) nach Anspruch 11, wobei distale Abschnitte der Seitenwände zu der Innenfläche zusammenlaufen.

14. Koaxialkanülenanordnung (20) nach Anspruch 10, wobei der Infusionsschlauch (40) mit der oberen Fläche des Stators verbunden ist.

15. Koaxialkanülenanordnung (20) nach Anspruch 10, wobei der Stator innerhalb des Reservoirs positioniert ist, sodass ein vergleichsweise konstanter Druck innerhalb des Reservoirs gehalten wird und wobei der Stator dazu konfiguriert ist, die Blutströmung um das Infusionsrohr (40) und in das Drainagerohr (52) zu führen, um die Rezirkulation innerhalb des Reservoirs zu reduzieren.

## Revendications

1. Un ensemble de canule coaxiale (20) comprenant :
un tube d'infusion (40) définissant une lumière de retour (46) et ayant une extrémité proximale (42) et une extrémité distale (44), dans lequel ladite extrémité distale (44) dudit tube d'infusion (40) comprend une pluralité d'orifices d'infusion (50), dans lequel ladite extrémité distale (44) dudit tube d'infusion (40) comprend un élément de restriction d'écoulement (122) positionné entre la pluralité d'orifices d'infusion (50) et une ouverture de retour terminale (48) située à une extrémité distale du tube d'infusion (40) ;
dans lequel l'élément de restriction d'écoulement (122) comprend un anneau définissant une lumière centrale, et dans lequel l'anneau crée une zone proximale de pression plus élevée dans la lumière de retour (46) qui augmente un débit à travers la pluralité d'orifices d'infusion (50) sans empêcher l'écoulement de fluide vers l'ouverture de retour terminale (48) ; et
un tube de drainage (52) coaxialement aligné avec ledit tube d'infusion (40) et ayant une extrémité proximale (70) et une extrémité distale (56), dans lequel ladite extrémité distale dudit tube de drainage (52) comprend une pluralité d'orifices de drainage (60) et dans lequel une longueur dudit tube de drainage (52) est inférieure à une longueur dudit tube d'infusion (40) ;
dans lequel une lumière de drainage (58) est définie par un espace entre ledit tube d'infusion (40) et ledit tube de drainage (52).

2. L'ensemble de canule coaxiale (20) selon la revendication 1, dans lequel l'élément de restriction d'écoulement (122) est positionné distalement par rapport au plus distal de la pluralité d'orifices d'infusion (50).

3. L'ensemble de canule coaxiale (20) selon la revendication 1, dans lequel la lumière centrale définit une surface de section transversale qui est plus petite qu'une surface de section transversale de l'ouverture de retour terminale (48).

4. L'ensemble de canule coaxiale (20) selon la revendication 1,
dans lequel une surface totale de section transversale des orifices d'infusion (50) est inférieure à une surface de section transversale du tube d'infusion (40).

5. L'ensemble de canule coaxiale (20) selon la revendication 1, dans lequel ladite extrémité distale (44) dudit tube d'infusion (40) comprend une pluralité d'éléments allongés (114) adjacents auxdits orifices d'infusion (50) et définissant l'ouverture de retour terminale (48) à une extrémité distale du tube d'infusion (40), et dans lequel ladite pluralité d'éléments allongés (114) définissent entre eux des fentes d'écoulement sanguin allongées axialement et ouvertes aux extrémités.

6. L'ensemble de canule coaxiale (20) selon la revendication 5, dans lequel la pluralité d'éléments allongés (114) est régulièrement espacée autour d'une circonférence de l'extrémité distale (44) du tube d'infusion (40).

7. L'ensemble de canule coaxiale (20) selon la revendication 6, dans lequel des éléments allongés (114) adjacents de ladite pluralité sont séparés par une seule fente d'écoulement sanguin ouverte aux extrémités et s'étendant axialement.

8. L'ensemble de canule coaxiale (20) selon la revendication 5, dans lequel chacun de ladite pluralité d'éléments allongés (114) comprend une paire de parois latérales qui convergent à l'extrémité distale.

9. L'ensemble de canule coaxiale (20) selon la revendication 8, dans lequel ladite paire de parois latérales convergent à l'extrémité distale dans une direction circonférentielle de telle sorte que le diamètre de section transversale des éléments allongés (114) est constant.

10. L'ensemble de canule coaxiale (20) selon la revendication 1, comprenant en outre un répartiteur d'écoulement (62) s'étendant dans et fixé à ladite extrémité proximale (54) dudit tube de drainage (70) et ayant un réservoir (72) pour recevoir un fluide provenant de ladite extrémité proximale dudit tube de drainage (70), dans lequel ledit répartiteur d'écoulement comprend un stator (78, 78a, 78b) s'étendant dans ledit réservoir et connecté audit tube d'infusion (40), dans lequel ledit stator est configuré pour guider l'écoulement sanguin autour dudit tube d'infusion (40).

11. L'ensemble de canule coaxiale (20) selon la revendication 10, dans lequel le stator comprend une surface supérieure (82), une extrémité distale (84) et une paire de parois latérales (86).

12. L'ensemble de canule coaxiale (20) selon la revendication 11, dans lequel les parois latérales convergent pour former l'extrémité distale.

13. L'ensemble de canule coaxiale (20) selon la revendication 11, dans lequel des parties distales des parois latérales convergent vers la surface intérieure.

14. L'ensemble de canule coaxiale (20) selon la revendication 10, dans lequel le tube d'infusion (40) est couplé à la surface supérieure du stator.

15. L'ensemble de canule coaxiale (20) selon la revendication 10, dans lequel le stator est positionné à l'intérieur du réservoir de telle sorte qu'une pression relativement constante est maintenue à l'intérieur du réservoir et dans lequel le stator est configuré pour guider l'écoulement sanguin autour du tube d'infusion (40) et dans le tube de drainage (52) afin de réduire la recirculation à l'intérieur du réservoir.
